(19) Europäisches Patentamt
European Patent Office
Office européen des brevets

(11) Veröffentlichungsnummer: **0 158 922**
**A2**

(12) # EUROPÄISCHE PATENTANMELDUNG

(21) Anmeldenummer: 85103951.1

(22) Anmeldetag: 02.04.85

(51) Int. Cl.[4]: C 07 D 405/06
C 07 D 413/06, C 07 D 307/14
A 01 N 43/08

(30) Priorität: 13.04.84 DE 3413996

(43) Veröffentlichungstag der Anmeldung: 23.10.85 Patentblatt 85/43

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Weissmüller, Joachim, Dr.
Carl-Langhausstrasse 53
D-4019 Monheim(DE)

(72) Erfinder: Krämer, Wolfgang, Dr.
Am Eckbusch 39/45
D-5600 Wuppertal 1(DE)

(72) Erfinder: Berg, Dieter, Dr.
Gellertweg 27
D-5600 Wuppertal 1(DE)

(72) Erfinder: Reinecke, Paul, Dr.
Lessingstrasse 11
D-5090 Leverkusen 1(DE)

(72) Erfinder: Brandes, Wilhelm, Dr.
Eichendorffstrasse 3
D-5653 Leichlingen(DE)

(72) Erfinder: Hänssler, Gerd, Dr.
Heymannstrasse 40
D-5090 Leverkusen(DE)

(72) Erfinder: Lürssen, Klaus, Dr.
August-Kierspel-Strasse 151
D-5060 Bergisch-Gladbach 2(DE)

(54) **Tetrahydrofuran-2-ylmethylmine.**

(57) Tetrahydrofuran-2-ylmethylamine der allgemeinen Formel (I),

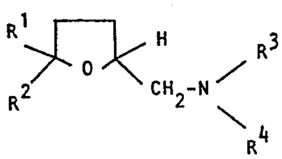

(I)

in welcher

$R^1$ für gegebenenfalls substituiertes Naphthyl, für gegebenenfalls substituiertes Cycloalkyl und Cycloalkenyl, für gegebenenfalls substituiertes Phenyl oder für durch - jeweils gegebenfalls substituiertes - Phenyl, Phenoxy oder Phenylthio substituiertes Alkyl steht,

$R^2$ für Wasserstoff oder Methyl steht,

$R^3$ für Alkyl steht und

$R^4$ für Alkyl, Alkenyl, Alkinyl oder gegebenenfalls substituiertes Arakyl steht oder

$R^3$ und $R^4$ gemeinsam mit dem Stickstoffatom, an das sie gebunden sind, für einen gegebenenfalls substituierten gesättigten Heterocyclus stehen, der weitere Heteroatome enthalten kann, ausgenommen die Verbindung, in der $R^3$ und $R^4$ beide für Methyl stehen, $R^1$ für unsubstituiertes Phenyl und $R^2$ gleichzeitig für Wasserstoff stehen,

sowie deren pflanzenverträgliche Säureadditionssalze und Metallsalzkomplexe, sowie ihre Verwendung als Pflanzenschutzmittel, vor allem als Fungizide und Pfflanzenwachstumsregulatoren.

Die neuen Tetrahydrofuran-2-yl-methylamine können z.B. hergestellt werden, wenn man geeignete Tetrahydrofuranderivate mit geeigneten Aminen umsetzt.

EP 0 158 922 A2

BAYER AKTIENGESELLSCHAFT
Konzernverwaltung RP
Patentabteilung

5090 Leverkusen, Bayerwerk
Bas/ABc
Ia

# Tetrahydrofuran-2-ylmethylamine

Die Erfindung betrifft neue Tetrahydrofuran-2-ylmethylamine, ein Verfahren zu ihrer Herstellung und ihre Verwendung als Pflanzenschutzmittel, vor allem als Fungizide und Pflanzenwachstumsregulatoren.

Es ist bereits bekannt, daß bestimmte 1,3-Dioxolan-4-yl-methylamine, wie beispielsweise das 2-/1-(3-Chlorphenoxy)-2-methylprop-2-yl7-2-methyl-4-(3-methylpiperidin-1-ylmethyl)-1,3-dioxolan oder das 2-/1-(2,4-Dichlorphenoxy)-2-methylprop-2-yl7-2-methyl-4-(3-methylpiperidin-1-ylmethyl)-1,3-dioxolan oder das 2-/1-(2,4-Dichlorphenoxy)-2-methylprop-2-yl7-2-methyl-4-(3,5-dimethylpiperidin-1-ylmethyl)-1,3-dioxolan, fungizide Eigenschaften besitzen (vgl. z.B. DE-OS 3 305 769).

Die fungiziden Eigenschaften dieser vorbekannten 1,3-Dioxolan-4-ylmethylamine können jedoch, insbesondere bei niedrigen Aufwandmengen und Konzentrationen, nicht immer in allen Anwendungsbereichen völlig zufriedenstellend sein.

Es wurden neue Tetrahydrofuran-2-ylmethylamine der allgemeinen Formel (I),

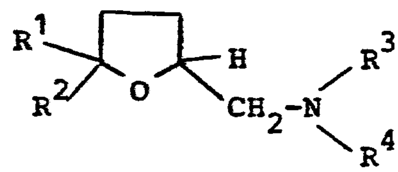

(I)

in welcher

$R^1$ für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes - Phenyl, Phenoxy oder Phenylthio, substituiertes Alkyl steht,

$R^2$ für Wasserstoff oder Methyl steht,

$R^3$ für Alkyl steht und

$R^4$ für Alkyl, Alkenyl, Alkinyl oder gegebenenfalls substituiertes Aralkyl steht oder

$R^3$ und $R^4$ gemeinsam mit dem Stickstoffatom, an das sie gebunden sind, für einen gegebenenfalls substituierten gesättigten Heterocyclus stehen, der weitere Heteroatome enthalten kann, ausgenommen die Verbindung, in der $R^3$ und $R^4$ beide für Methyl stehen, $R^1$ für unsubstituiertes Phenyl und $R^2$ gleichzeitig für Wasserstoff stehen,

sowie deren pflanzenverträgliche Säureadditionssalze und Metallsalzkomplexe gefunden.

Die Verbindungen der Formel (I) können als geometrische und/oder optische Isomere oder Isomerengemische unterschiedlicher Zusammensetzung anfallen. Sowohl die reinen Isomeren als auch die Isomerengemische werden erfindungsgemäß beansprucht.

Weiterhin wurde gefunden, daß man die neuen Tetrahydrofuran-2-ylmethylamine der allgemeinen Formel (I)

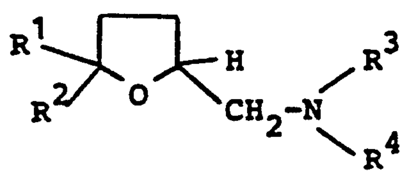

(I)

in welcher

$R^1$ für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes - Phenyl, Phenoxy oder Phenylthio substituiertes Alkyl steht,

$R^2$ für Wasserstoff oder Methyl steht,

$R^3$ für Alkyl steht und

$R^4$ für Alkyl, Alkenyl, Alkinyl oder gegebenenfalls substituiertes Aralkyl steht oder

$R^3$ und $R^4$ gemeinsam mit dem Stickstoffatom, an das sie gebunden sind, für einen gegebenenfalls substituierten gesättigten Heterocyclus stehen, der weitere Heteroatome enthalten kann,

ausgenommen die Verbindung, in der $R^3$ und $R^4$ beide für Methyl stehen, $R^1$ für unsubstituiertes Phenyl und $R^2$ gleichzeitig für Wasserstoff stehen, sowie deren pflanzenverträglichen Säureadditionssalze und Metallsalzkomplexe erhält, wenn man

Tetrahydrofuranderivate der Formel (II)

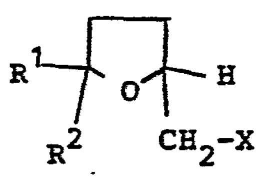

(II)

in welcher

$R^1$ und $R^2$ die oben angegebene Bedeutung haben und

X für eine elektronenanziehende Abgangsgruppierung steht,

mit Aminen der allgemeinen Formel (III)

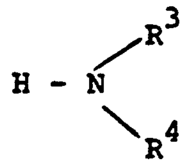

(III)

in welcher

$R^3$ und $R^4$ die oben angegebene Bedeutung haben,

gegebenenfalls in Gegenwart eines Verdünnungsmittels, gegebenenfalls in Gegenwart eines Katalysators und gegebenenfalls in Gegenwart eines Säurebindemittels umsetzt, und gegebenenfalls anschließend eine Säure oder ein Metallsalz addiert.

Außerdem ist es möglich, die erfindungsgemäßen Tetrahydrofuran-2-ylmethylamine der Formel (I) nach allgemein üblichen Methoden am Stickstoff zu den entsprechenden tetrasubstituierten Ammoniumsalzen zu quaternieren.

Schließlich wurde gefunden, daß die neuen Tetrahydrofuran-2-ylmethylamine der allgemeinen Formel (I) fungizide und pflanzenwachstumsregulierende Eigenschaften besitzen.

Dabei zeigen die erfindungsgemäßen Tetrahydrofuran-2-ylmethylamine der Formel (I) überraschenderweise eine höhere fungizide Wirksamkeit als die aus dem Stand der Technik bekannten 1,3-Dioxolan-4-ylmethylamine, wie

beispielsweise das 2/1-(3-Chlorphenoxy)-2-methylprop-2-yl7-2-methyl-4-(3-methylpiperidin-1-ylmethyl)-1,3-dioxolan oder das 2-/1-(2,4-Dichlorphenoxy)-2-methyl-prop-2-yl7-2-methyl-4-(3-methylpiperidin-1-ylmethyl)-1,3-dioxolan oder das 2-/1-(2,4-Dichlorphenoxy)-2-methyl-prop-2-yl7-2-methyl-4-(3,5-dimethylpiperidin-1-ylmethyl)-1,3-dioxolan, welches chemisch und wirkungsmäßig naheliegende Verbindungen sind.

Die erfindungsgemäßen Tetrahydrofuran-2-ylmethylamine sind durch die Formel (I) allgemein definiert. Bevorzugt sind Verbindungen der Formel (I), bei welchen

$R^1$ für gegebenenfalls einfach oder mehrfach, gleich oder verschieden substituiertes Naphthyl steht, wobei als Substituenten in Frage kommen: Hydroxy, Halogen, jeweils geradkettiges oder verzweigtes Alkyl, Alkoxy, Alkenyloxy, Alkinyloxy oder Alkanoyloxy mit je bis zu 4 Kohlentoffatomen in den Alkylteilen; weiterhin für jeweils gegebenenfalls einfach oder mehrfach, gleich oder verschieden durch geradkettiges oder verzweigtes Alkyl mit 1 bis 4 Kohlenstoffatomen substituiertes Cycloalkyl oder Cycloalkenyl mit je 3 bis 7 Kohlenstoffatomen steht; für gegebenenfalls einfach oder mehrfach, gleich oder verschieden substituiertes Phenyl oder für im Phenylkern gegebenenfalls einfach oder mehrfach, gleich oder verschieden substituiertes Phenylalkyl, Phenoxyalkyl oder Phenylthioalkyl mit jeweils bis zu 8 Kohlenstoffatomen im geradkettigen oder verzweigten Alkylteil steht, wobei als Phenylsubstituenten jeweils in Frage kommen: Hydroxy, Halogen, Cyano, Nitro, jeweils geradkettiges oder verzweigtes Alkyl, Alkoxy, Alkenyloxy, Alkinyloxy und Alkylthio mit jeweils bis zu 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Cycloalkyl mit 5 bis 7 Kohlenstoffatomen, geradkettiges oder verzweigtes Alkoxycarbonyl bzw. Alkanoyloxy mit jeweils bis zu 4 Kohlenstoffatomen im Alkylteil, gegebenenfalls durch Halogen, insbesondere Fluor oder Chlor, oder durch geradkettiges oder verzweigtes Alkyl mit 1 bis 4 Kohlenstoffatomen substituiertes Phenyl oder Phenoxy oder der Rest R-O-N=CH-, wobei R jeweils für geradkettiges oder verzweigtes Alkyl, Alkenyl oder Alkinyl mit bis zu 4 Kohlenstoffatomen steht;

$R^2$ für Wasserstoff oder Methyl steht,

$R^3$ für geradkettiges oder verzweigtes Alkyl mit 1 bis 4 Kohlenstoffatomen steht und

$R^4$ für geradkettiges oder verzweigtes Alkyl mit 1 bis 6 Kohlenstoffatomen, für geradkettiges oder verzweigtes Alkenyl oder Alkinyl mit jeweils bis zu 6 Kohlenstoffatomen oder für gegebenenfalls einfach oder mehrfach, gleich oder verschieden substituiertes Aralkyl mit 1 oder 2 Kohlenstoffatomen im Alkylteil und 6 bis 10 Kohlenstoffatomen im Arylteil

steht, wobei als Arylsubstituenten jeweils in Frage kommen: Halogen, geradkettiges oder verzweigtes Alkyl mit 1 bis 4 Kohlenstoffatomen oder Halogenalkyl mit 1 oder 2 Kohlenstoffatomen und 3 bis 5 gleichen oder verschiedenen Halogenatomen,

$R^3$ und $R^4$ zusammen mit dem Stickstoffatom, an welches sie gebunden sind, für einen gegebenenfalls einfach oder mehrfach, gleich oder verschieden substituierten 5- bis 7-gliedrigen gesättigten Heterocyclus mit 1 bis 3 Heteroatomen, vorzugsweise Stickstoff und Sauerstoff stehen, wobei als Substituenten in Frage kommen: geradkettiges oder verzweigtes Alkyl mit 1 bis 4 Kohlenstoffatomen, geradkettiges oder verzweigtes Alkoxycarbonyl mit bis zu 5 Kohlenstoffatomen, Phenyl, Hydroxymethyl sowie die $R'-CO-O-CH_2$-Gruppe, wobei R' für geradkettiges oder verzweigtes Alkyl, Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6 Kohlenstoffatomen, in beiden Alkylteilen steht, wobei jedoch $R^3$ und $R^4$ nicht beide für Methyl stehen, wenn $R^1$ für unsubstituiertes Phenyl steht und gleichzeitig $R^2$ für Wasserstoff steht.

Besonders bevorzugt sind Verbindungen der allgemeinen Formel (I), bei denen

$R^1$ für gegebenenfalls ein- bis dreifach, gleich oder verschieden substituiertes Naphthyl steht, wobei als Substituenten in Frage kommen: Fluor, Chlor, Brom, Hydroxy, Methyl, Ethyl, Methoxy, Ethoxy, Allyloxy, Propargyloxy und Acetyloxy;

weiterhin für jeweils gegebenenfalls ein- bis dreifach, gleich oder verschieden durch Methyl oder Ethyl substituiertes Cycloalkyl oder Cycloalkenyl mit 5 bis 7 Kohlenstoffatomen, für gegebenenfalls ein- bis dreifach, gleich oder verschieden substituiertes Phenyl oder für einen im Phenylkern gegebenenfalls ein- bis dreifach, gleich oder verschieden substituierten Rest der Formel

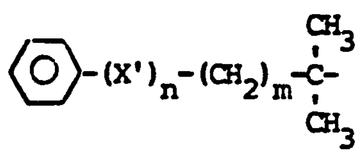 , 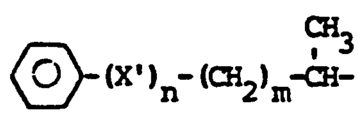 ,

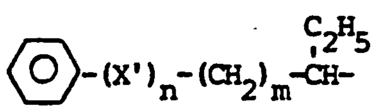 oder 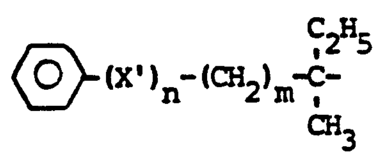

steht, wobei als Phenylsubstituenten jeweils in Frage kommen: Hydroxy, Fluor, Chlor, Brom, Cyano, Nitro, Methyl, Methoxy, Methylthio, Ethyl, Ethoxy, Ethylthio, n- und i-Propyl, Isopropyloxy, n-, i-,

s- und t-Butyl, Allyloxy, Propargyloxy, Trifluormethyl, Trifluormethoxy, Trifluormethylthio, Cyclohexyl, Methoxycarbonyl, Ethoxycarbonyl, Acetyloxy, gegebenenfalls durch Fluor, Chlor oder Methyl substituiertes Phenyl oder Phenoxy, sowie der Rest R-O-N=CH-, wobei R jeweils für Methyl, Ethyl, n- und i-Propyl, n-, i-, s- und t-Butyl, Allyl und Propargyl steht; wobei in allen oben formelmäßig dargestellten Resten X' jeweils für Sauerstoff oder Schwefel, n für 0 oder 1 und m ebenfalls für 0 oder 1 steht,

$R^2$ für Wasserstoff oder Methyl steht,

$R^3$ für Methyl, Ethyl, n- und i-Propyl steht und

$R^4$ für Methyl, Ethyl, n- und i-Propyl, n-, i-, s- und t-Butyl, Neopentyl, Allyl, 2-Butenyl, 3-Methyl-2-butenyl, Propargyl, sowie für gegebenenfalls ein- bis dreifach, gleich oder verschieden durch Fluor, Chlor, Methyl oder Trifluormethyl substituiertes Benzyl steht oder

$R^3$ und $R^4$ zusammen mit dem Stickstoffatom, an welches sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl oder 1-Perhydroazepinyl stehen, wobei als Substituenten genannt seien: Methyl, Ethyl, n- und i-Propyl, Phenyl, Hydroxymethyl, Acetyloxymethyl, Methoxycarbonyloxymethyl, Dimethylaminocarbonyloxymethyl, Diethylaminocarbonyloxymethyl oder Methoxyacetyloxymethyl, ausgenommen die Verbindung in der $R^3$ und $R^4$ für Methyl, $R^1$ für unsubstituiertes Phenyl und $R^2$ gleichzeitig für Wasserstoff stehen.

Im einzelnen seien außer den bei den Herstellungsbeispielen genannten Verbindungen die folgenden Verbindungen der allgemeinen Formel (I) genannt:

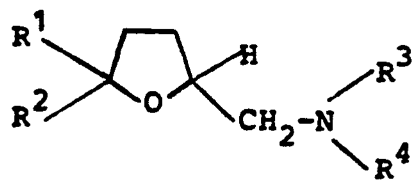

(I)

| $R^1$ | $R^2$ | 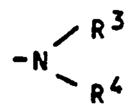 |
|---|---|---|
| 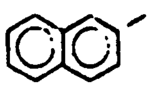 | $CH_3$ | 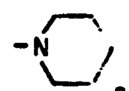 |
| 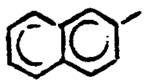 | $CH_3$ | 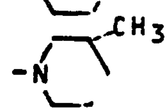 |
| 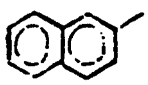 | $CH_3$ | 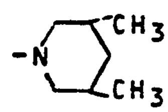 |
| 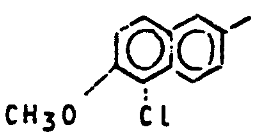 | $CH_3$ | 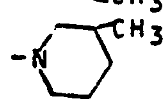 |
| 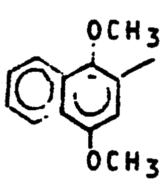 | $CH_3$ | 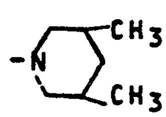 |

| $R^1$ | $R^2$ | 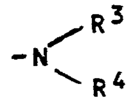 |
|---|---|---|
| 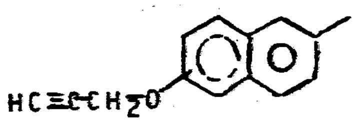 | $CH_3$ | 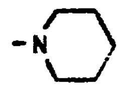 |
| 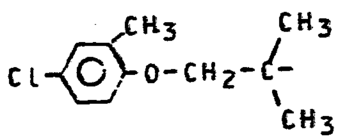 | $CH_3$ | 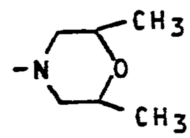 |
| 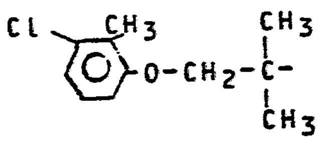 | $CH_3$ | 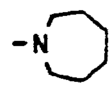 |
| 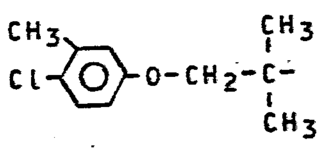 | $CH_3$ | 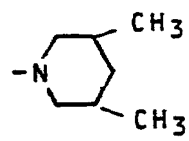 |
| 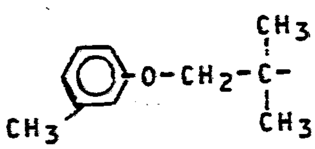 | $CH_3$ | 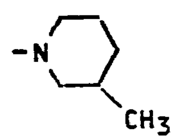 |
| 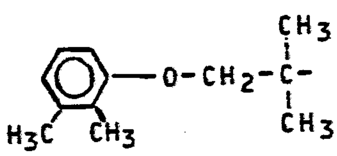 | $CH_3$ | 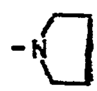 |
| 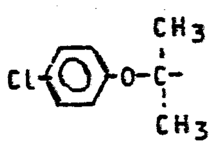 | $CH_3$ | 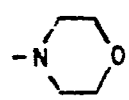 |

| $R^1$ | $R^2$ | 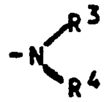 |
|---|---|---|
| 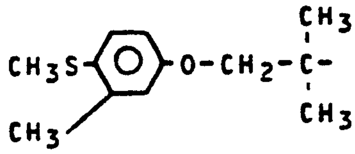 | $CH_3$ | 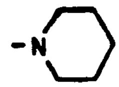 |
| 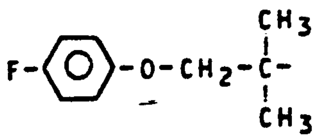 | $CH_3$ | 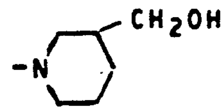 |
| 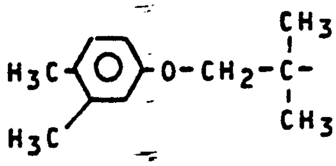 | $CH_3$ | 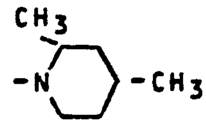 |
| 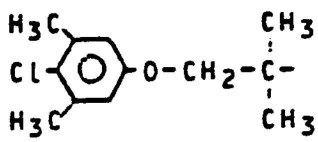 | $CH_3$ | 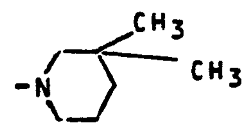 |
| 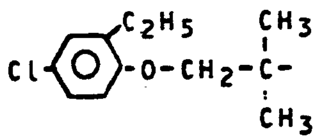 | $CH_3$ | 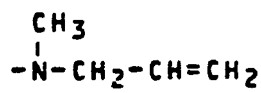 |
| 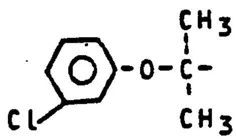 | $CH_3$ | 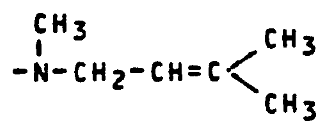 |
| 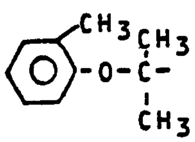 | $CH_3$ | 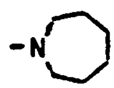 |
| 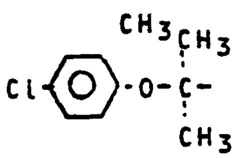 | $CH_3$ | 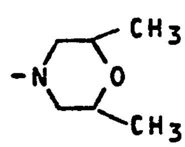 |
| 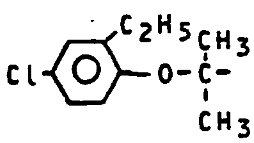 | $CH_3$ | 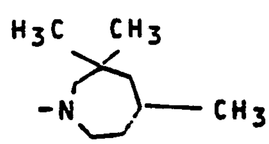 |
| 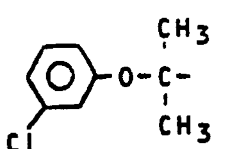 | $CH_3$ | 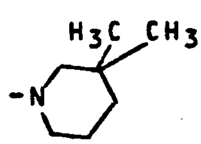 |
| 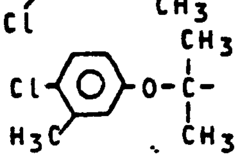 | $CH_3$ | 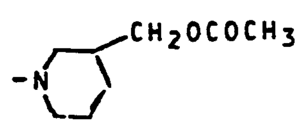 |

| $R^1$ | $R^2$ | 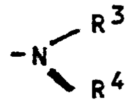 |
|---|---|---|
| 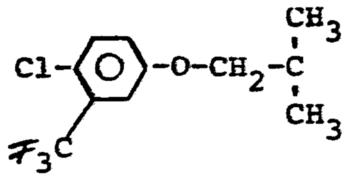 | $CH_3$ | 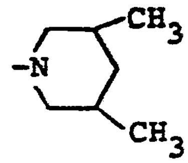 |
| 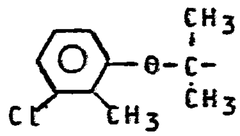 | $CH_3$ | 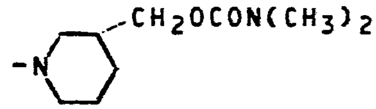 |
| 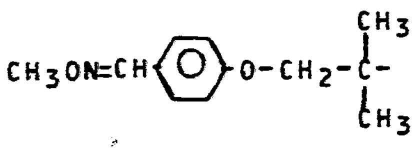 | $CH_3$ | 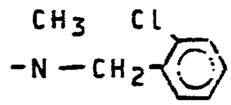 |
| 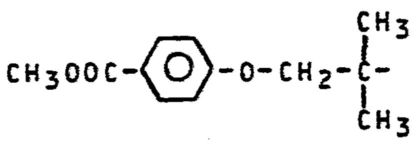 | $CH_3$ | 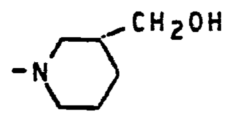 |
| 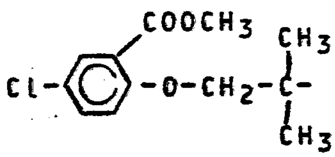 | $CH_3$ | 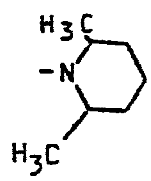 |
| 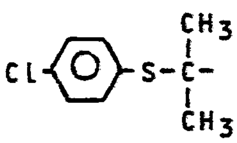 | $CH_3$ | 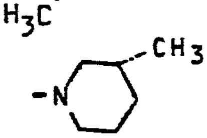 |
| 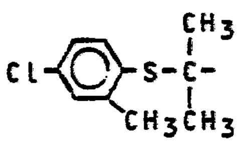 | $CH_3$ | 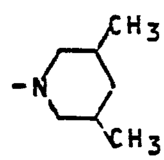 |
| 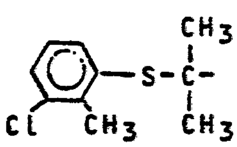 | $CH_3$ | 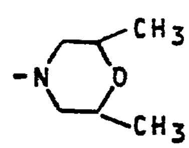 |
| 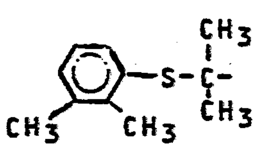 | $CH_3$ | 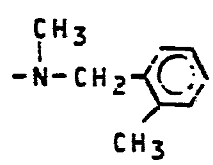 |

| $R^1$ | $R^2$ | 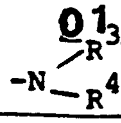 |
|---|---|---|
| 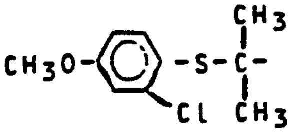 | $CH_3$ | 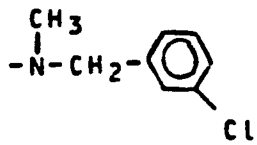 |
| 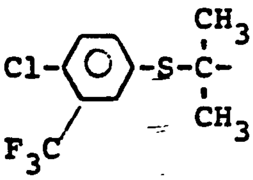 | $CH_3$ | 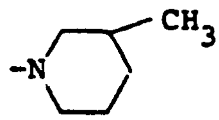 |
| 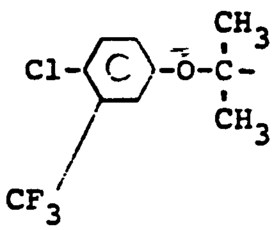 | $CH_3$ | 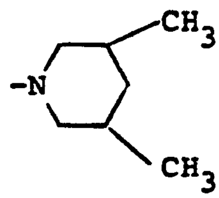 |
| 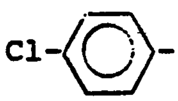 | $CH_3$ | 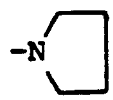 |
| 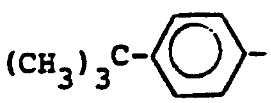 | $CH_3$ | 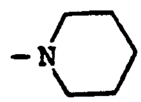 |
| 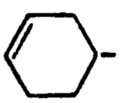 | H | 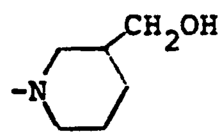 |
| 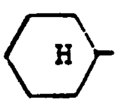 | $CH_3$ | 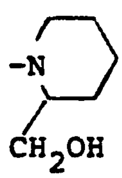 |

| $R^1$ | $R^2$ | 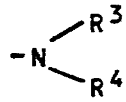 |
|---|---|---|
| 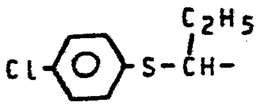 | $CH_3$ | 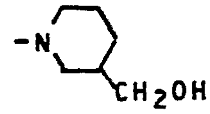 |
| 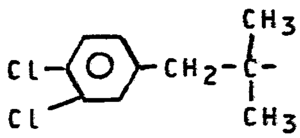 | $CH_3$ | 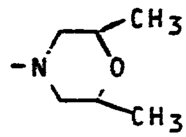 |
| 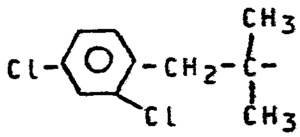 | $CH_3$ | 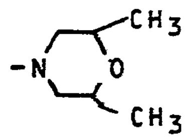 |
| 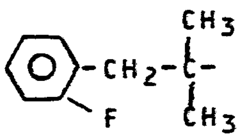 | $CH_3$ | 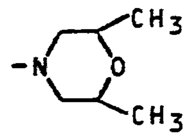 |
| 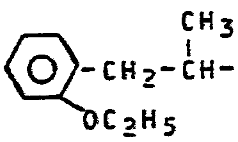 | $CH_3$ | 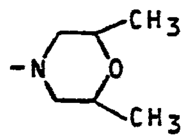 |
| 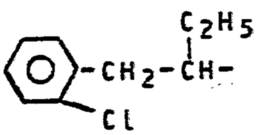 | $CH_3$ | 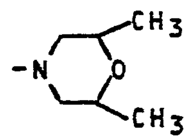 |
| 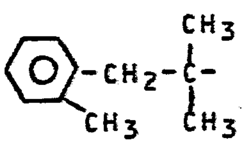 | $CH_3$ | 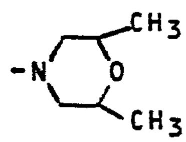 |
| 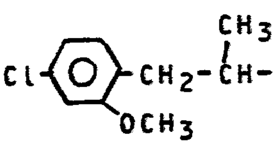 | $CH_3$ | 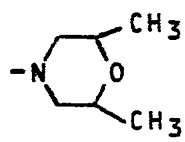 |
| 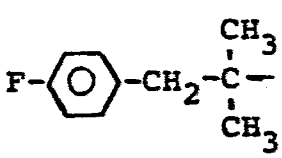 | $CH_3$ | 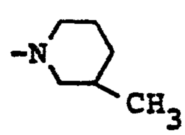 |

Verwendet man als Ausgangsstoffe beispielsweise 2-Brommethyl-5-(4-chlorphenyl)-tetrahydrofuran und 3,5-Dimethylpiperidin, so läßt sich der Reaktionsablauf des erfindungsgemäßen Verfahrens durch das folgende Formelschema darstellen:

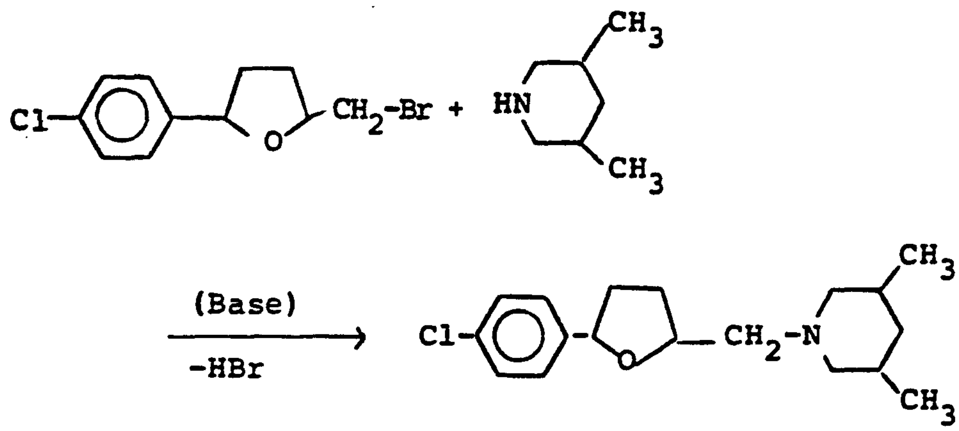


Die zur Durchführung des erfindungsgemäßen Verfahrens als Ausgangsstoffe benötigten Tetrahydrofuranderivate sind durch die Formel (II) allgemein definiert. In dieser Formel (II) stehen $R^1$ und $R^2$ vorzugsweise für diejenigen Substituenten, die schon bei der Beschreibung der erfindungsgemäßen Stoffe der Formel (I) als bevorzugt für diese Reste genannt wurden.

X steht vorzugsweise für Halogen, insbesondere Chlor, Brom oder Jod, oder für gegebenenfalls substituiertes Alkylsulfonyloxy, Alkoxysulfonyloxy oder Arylsulfonyloxy, insbesondere für Methansulfonyloxy, Methoxysulfonyloxy, Trifluormethansulfonyloxy oder p-Toluolsulfonyloxy.

Die Tetrahydrofuranderivate der Formel (II) sind teilweise bekannt (vgl. z.B. EP-OS 68 331). Man erhält sie beispielsweise, wenn man Alkenylcarbinole der Formel (IV)

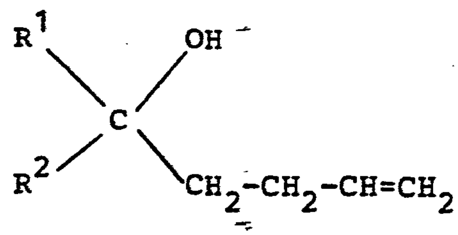

(IV)

in welcher

$R^1$ und $R^2$ die oben angegebene Bedeutung haben,

mit Halogen, wie beispielsweise Brom oder Jod, gegebenenfalls in Gegenwart eines Verdünnungsmittels, wie beispielsweise Diethylether, und gegebenenfalls in Gegenwart eines Katalysators oder Säurebindemittels, wie Kaliumiodid, Chinolin oder Kaliumcarbonat, bei Temperaturen zwischen +20°C und 120°C umsetzt (vgl. z.B. Houben-Weyl "Methoden der organischen Chemie" Band VI/3, S. 537, 4. Auflage, Thieme Verlag Stuttgart; Ukr. Khim. Zh. 48, 72-76 (1982)), oder

wenn man Trihydroxyverbindungen der Formel (V)

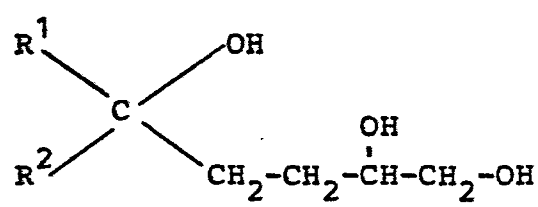

(V)

in welcher

$R^1$ und $R^2$ die oben angegebene Bedeutung haben,

mit Säuren, wie beispielsweise Schwefelsäure oder Phosphorsäure, in üblicher Weise cyclisiert (vgl. z.B. Houben-Weyl "Methoden der organischen Chemie" Band VI/3 S.528; 4. Auflage, Thieme Verlag Stuttgart oder Khim. Geterotsikl. Soedin. Sb. No. 2, 15-17, /1970/ bzw. C.A. 77, 48114 g) und die so erhältlichen Hydroxymethyltetrahydrofurane der Formel (VI)

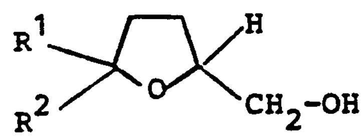

(VI)

in welcher

$R^1$ und $R^2$ die oben angegebene Bedeutung haben,

in einer 2. Stufe beispielsweise mit Sulfonsäurehalogeniden der Formel (VII)

$$R^5-SO_2-Hal \quad (VII)$$

in welcher

$R^5$ für gegebenenfalls substituiertes Alkyl, Alkoxy oder Aryl, insbesondere für Methyl, Methoxy, Trifluormethyl oder p-Tolyl steht, und

Hal für Halogen, insbesondere Chlor oder Brom steht,

gegebenenfalls in Gegenwart eines Verdünnungsmittels, wie beispielsweise Dichlormethan, und gegebenenfalls in Gegenwart eines Säurebindemittels, wie beispielsweise Pyridin, bei Temperaturen zwischen 0°C und +120°C umsetzt.

Die Alkenylcarbinole der Formel (IV) und die Trihydroxyverbindungen der Formel (VI) sind bekannt oder lassen sich nach bekannten Verfahren in einfacher, analoger Weise herstellen (vgl. z.B. Bull. Soc. Chim. France 1967, 2466-2472, Helvetica. Chim. Acta 64, 2606-2613 [1981], Tetrahedron Lett. 22, 4995-4998 [1981]).

Die Sulfonsäurehalogenide der Formel (VII) sind allgemein bekannte Verbindungen der organischen Chemie.

Die weiterhin zur Durchführung des erfindungsgemäßen Verfahrens als Ausgangsstoffe benötigten Amine sind durch die Formel (III) allgemein definiert. In dieser Formel (III) stehen $R^3$ und $R^4$ vorzugsweise für diejenigen Reste, die schon bei der Beschreibung der erfindungsgemäßen Stoffe der Formel (I) als bevorzugt für diese Substituenten genannt werden.

Die Amine der Formel (III) sind allgemein bekannte Verbindungen der organischen Chemie.

Als Verdünnungsmittel zur Durchführung des erfindungsgemäßen Verfahrens kommen inerte organische Lösungsmittel in Frage.

Hierzu gehören insbesondere aliphatische oder aromatische gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Dioxan, Tetrahydrofuran oder Ethylenglykoldimethyl- oder -diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester oder Sulfoxide, wie Dimethylsulfoxid.

Das erfindungsgemäße Verfahren kann gegebenenfalls in Gegenwart eines Säurebindemittels durchgeführt werden.

Als solche kommen alle üblichen anorganischen oder organischen Basen in Frage. Hierzu gehören beispielsweise Alkalimetallhydroxide, wie Natriumhydroxid oder Kaliumhydroxid; Alkalimetallcarbonate, wie Natriumcarbonat, Kaliumcarbonat oder Natriumhydrogencarbonat; sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Es ist auch möglich, das als jeweiligen Reaktionspartner eingesetzte Amin der Formel (III) im entsprechenden Überschuß als Säurebindemittel zu verwenden.

Das erfindungsgemäße Verfahren wird gegebenenfalls in Gegenwart eines Katalysators durchgeführt. Vorzugsweise verwendet man Alkalimetalliodide, wie zum Beispiel Kaliumiodid.

Die Reaktionstemperaturen können bei der Durchführung des erfindungsgemäßen Verfahrens in einem größeren Bereich variiert werden. Im allgemeinen arbeitet man bei Temperaturen zwischen +20°C und +250°C, vorzugsweise bei Temperaturen zwischen +50°C und +200°C.

Das erfindungsgemäße Verfahren kann bei Normaldruck, aber auch bei erhöhtem Druck durchgeführt werden. Unter Druck arbeitet man im allgemeinen zwischen etwa 1,5 atü und 5 atü, vorzugsweise zwischen 1,5 atü und 3 atü.

Bei der Durchführung des erfindungsgemäßen Verfahrens setzt man pro Mol Tetrahydrofuranderivat der Formel (II) im allgemeinen 1 bis 30 Mol, vorzugsweise 1 bis 15 Mol, Amin der Formel (III) und gegebenenfalls 1 bis 5 Mol an Säurebindemittel und gegebenenfalls 0,01 bis 0,5 Mol an Katalysator ein.

Die Aufarbeitung und Isolierung der Endprodukte der Formel (I) erfolgt nach allgemein üblichen Verfahren.

Zur Herstellung von pflanzenverträglichen Säureadditionssalzen der Formel (I) kommen vorzugsweise folgende Säuren in Frage: Halogenwasserstoffsäuren, wie z.B. Chlorwasserstoffsäure und Bromwasserstoffsäure, insbesondere Chlorwasserstoffsäure, ferner Phosphorsäure,

Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten: Halogenwasserstoffsäuren, wie z.B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol, und Hinzufügen zur Verbindung der Formel (I). Man kann Metallsalz-Komplexe in bekannter Weise, z.B. durch Abfiltrieren, Isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz wurden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die aufgezählten Oberbegriffe fallen, genannt:

Botrytis-Arten, wie beispielsweise Botrytis cinera;
Plasmopara-Arten, wie beispielsweise Plasmopara viticola;
Uromyces-Arten, wie beispielsweise Uromyces appendiculatus;
Sphaerotheca-Arten, wie beispielsweise Sphaerotheca fuliginea,
Venturia-Arten, wie beispielsweise Venturia inaequalis;
Podosphaera-Arten, wie beispielsweise Podosphaera leucotricha;

Phytophthora-Arten, wie beispielsweise Phytophthora infestans;
Erysiphe-Arten, wie beispielsweise Erysiphe graminus;
Puccinia-Arten, wie beispielsweise Puccinia recondita;
Fusarium-Arten, wie beispielsweise Fusarium culmorum;
Ustilago-Arten, wie beispielsweise Ustilago nuda oder Ustilago avenae;
Septoria-Arten, wie beispielsweise Septoria nodorum;
Tilletia-Arten, wie beispielsweise Tilletia caries;
Pyricularia-Arten, wie beispielsweise Pyricularia oryzae;
Pellicularia-Arten, wie beispielsweise Pellicularia sasakii;
Pyrenophora-Arten, wie beispielsweise Pyrenophora teres
(Konidienform: Drechslera, Syn: Helminthosporium);
Cochliobolus-Arten, wie beispielsweise Cochliobolus sativus
(Konidienform: Drechslera, Syn: Helminthosporium);
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des Braunrostes am Weizen (Puccinia recondita) gegen den Erreger der Braunfleckenkrankheit des Weizens (Septoria nodorum), gegen den Erreger der Blattfleckenkrankheit

der Gerste (Pyrenophora teres) oder gegen Mehltauerreger, zur Bekämpfung von Reiskrankheiten, wie beispielsweise gegen den Erreger der Reisfleckenkrankheit (Pyricularia oryzae) oder zur Bekämpfung von Gemüsekrankheiten, wie beispielsweise gegen den Erreger der Tomatenbraunfäule (Phytophthora infestans) einsetzen. Sie zeigen neben einer hervorragenden protektiven Wirksamkeit auch sehr gute systemische Eigenschaften und in-vitro Wirksamkeit im Agarplattentest.

Außerdem greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch

der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Sattgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispersionsmitteln und/oder schaumerzeugenden Mitteln. Im Falle der Benutzung von Wasser als Streckmittel können z.B.

auch organische Lösungsmittel als Hilfslösungsmittel verwendet werden. Als flüssige Lösungsmittel kommen im wesentlichen in Frage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether,

z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Für die Verwendung als Fungizide gilt:

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Aufwandmengen können bei der Verwendung als Wachstumsregulatoren in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Herstellungsbeispiele

Beispiel 1:

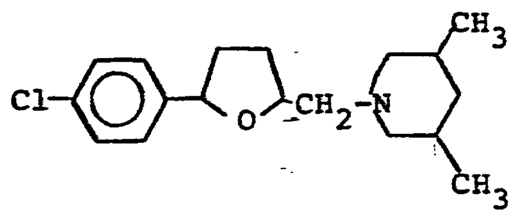

20 g (0,073 Mol) 2-Brommethyl-5-(4-chlorphenyl)-tetrahydrofuran und 25 g (0,22 Mol) 3,5-Dimethylpiperidin werden für 16 Stunden auf 120°C erhitzt. Die erkaltete Reaktionsmischung wird in Essigsäureethylester aufgenommen, zweimal mit Wasser gewaschen, über Natriumsulfat getrocknet und im Vakuum eingeengt. Der ölige Rückstand wird säulenchromatographisch (Kieselgel 60/Ligroin-Essigester 2:1/Essigester) gereinigt. Man erhält 4,5 g (20 % der Theorie) an 2-(4-Chlorphenyl)-5-(3,5-dimethylpiperidin-1-ylmethyl)-tetrahydrofuran als Öl; $n_D^{20}$ = 1,5159.

Herstellung des Ausgangsstoffes:

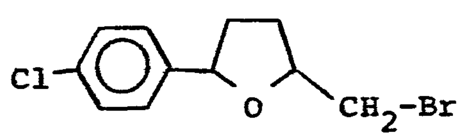

17,3 g (0,1 Mol) 5-(4-Chlorphenyl)-pent-1-en-5-ol in 50 ml absolutem Ether werden bei 0°C tropfenweise mit 16 g (0,1 Mol) Brom versetzt. Nach beendeter Zugabe gibt man 13 g (0,1 Mol) Chinolin zu, wobei die Temperatur auf 15°C ansteigt und Chinolinhydrobromid ausfällt. Der Niederschlag wird abgesaugt, das Filtrat im Vakuum eingedampft und der Rückstand eine Stunde im Wasserbad erhitzt. Nach dem Abkühlen nimmt man in Ether auf, wäscht mit 15 prozentiger Salzsäure und mit Wasser, trocknet über Natriumsulfat, engt im Vakuum ein und destilliert den Rückstand im Hochvakuum. Man erhält 5 g (18 % der Theorie) an 2-Brommethyl-5-(4-chlorphenyl)-tetrahydrofuran vom Siedepunkt 145°C / 0,13 mbar.

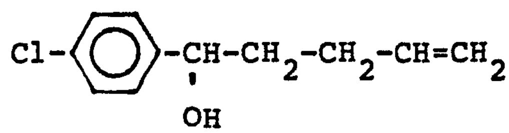

Zu einer Grignard-Lösung aus 12,2 g (0,5 Mol) Magnesium und 67,5 g (0,5 Mol) 4-Brom-1-buten in 350 ml absolutem Ether tropft man unter Rühren eine Lösung von 70 g (0,5 Mol) 4-Chlorbenzaldehyd in 100 ml absolutem Ether und erhitzt nach beendeter Zugabe für 2 Stunden auf Rückflußtemperatur. Die abgekühlte Reaktionsmischung wird in eine Mischung aus 1 l gesättigter Ammoniumchloridlösung und Eis gegossen, die organische Phase abgetrennt, zweimal mit Wasser gewaschen, über Natriumsulfat getrocknet, im Vakuum eingeengt und anschließend im Hochvakuum destilliert. Man erhält 50 g (58 % der Theorie) an 5-(4-Chlorphenyl)-pent-1-en-5-ol vom Siedepunkt 100°C-105°C/ 0,13 mbar.

Beispiel 2:

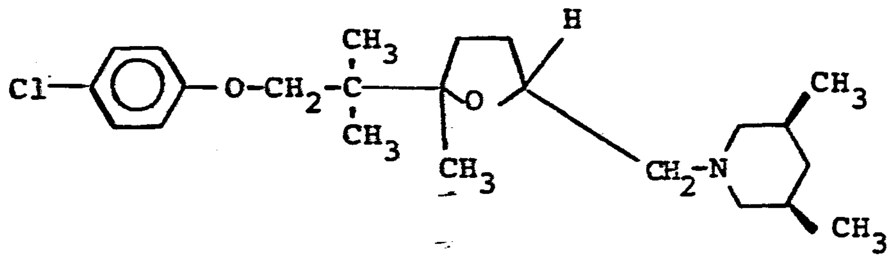

16 g (0,044 Mol) 2-Brommethyl-5-/1-(4-chlorphenoxy)-2-methylprop-2-yl7-5-methyl-tetrahydrofuran werden zusammen mit 11 g (0,097 Mol) cis-3,5-Dimethylpiperidin circa 14 Stunden bei einer Badtemperatur von 140°C gerührt. Die erhaltene Reaktionsmischung wird in Ether aufgenommen, mehrfach mit Wasser gewaschen, über Natriumsulfat getrocknet und im Vakuum eingeengt. Der ölige Rückstand wird säulenchromatographisch (Kieselgel 60 Ether-Petrolether 1:1) gereinigt. Man erhält 7,8 g (45 % der Theorie) an 5-/1-(4-Chlorphenoxy)-2-methylprop-2-yl7-2-(cis)-(3,5-dimethylpiperidin-1-ylmethyl)-5-methyl-tetrahydrofuran vom Brechungsindex $n_D^{20}$: 1.5049.

Herstellung der Ausgangsverbindung

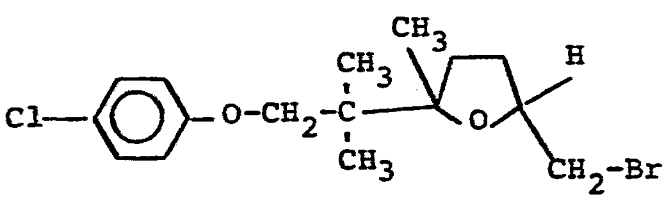

28,2 g (0,1 Mol) 7-(4-Chlorphenoxy)-5-hydroxy-5,6,6-trimethyl-hept-1-en in 200 ml absolutem Chloroform werden unter Rühren bei Raumtemperatur tropfenweise mit 16 g (0,1 Mol) Brom und danach bei -10°C unter Kühlung mit 13 g (0,1 Mol) Chinolin versetzt. Die Reaktionsmischung wird weitere 2 Stunden bei Raumtemperatur gerührt, im Vakuum eingeengt, in Ether aufgenommen, filtriert, wiederum im Vakuum eingeengt und 1 Stunde im Wasserbad bei 95°C gerührt. Die erhaltene Mischung wird in Ether aufgenommen, filtriert, mit 15 prozentiger Salzsäure und danach mit Wasser gewaschen, über Natriumsulfat getrocknet und im Vakuum vom Lösungsmittel befreit. Man erhält 32,7 g (90 % der Theorie) an 2-Brommethyl-5-/1-(4-chlorphenoxy)-2-methylprop-2-yl/-5-methyl-tetrahydrofuran als Öl, welches ohne weitere Reinigung in die nächste Stufe eingesetzt werden kann.

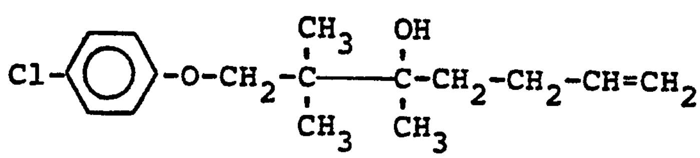

Zu einer Grignard-Lösung aus 7,2 g (0,3 Mol) Magnesium und 36 g Allylbromid in 300 ml absolutem Ether tropft man unter Rühren und Eiskühlung eine Lösung von 48 g (0,2 Mol) 2-/1-(4-Chlorphenoxy)-2-methylprop-2-yl/-2-methyl-oxiran in 100 ml absolutem Tetrahydrofuran, erhitzt nach beendeter Zugabe für 4 Stunden auf Rückflußtemperatur und rührt weitere 15 Stunden bei Raumtemperatur nach. Zur Aufarbeitung hydrolysiert man mit wäßriger Ammoniumchloridlösung, trennt die organische Phase ab, trocknet über Natriumsulfat, entfernt das Lösungsmittel im Vakuum und destilliert den Rückstand im Hochvakuum.

Man erhält 43 g (51 % der Theorie) an 7-(4-Chlorphenoxy)-5-hydroxy-5,6,6-trimethyl-hept-1-en vom Siedepunkt 128°-130°C/0,13 mbar.

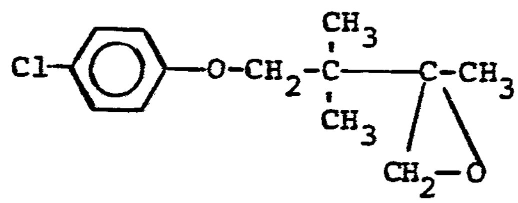

Eine Suspension von 72 g (0,33 Mol) Trimethylsulfoxoniumiodid in 71 g (0,3 Mol) absolutem Dimethylsulfoxid werden innerhalb von 10 Minuten mit 70,2 g (0,33 Mol) Natriummethylat versetzt, dann mit 100 ml absolutem Tetrahydrofuran verdünnt, drei Stunden bei Raumtemperatur nachgerührt und dann tropfenweise mit 68 g (0,3 Mol) 1-(4-Chlorphenoxy)-2,2-dimethyl-butan-3-on in 50 ml absolutem Tetrahydrofuran versetzt. Nach beendeter Zugabe rührt man 2 Tage bei Raumtemperatur, filtriert den ausgefallenen Feststoff ab, engt das Filtrat im Vakuum ein, löst den Rückstand in 300 ml Dichlormethan, wäscht mehrmals mit insgesamt 200 ml Wasser, trocknet über Natriumsulfat und entfernt das Lösungsmittel im

Vakuum. Man erhält 62 g (86 % der Theorie) an 2-/1-(4-Chlorphenoxy)-2-methyl-prop-2-yl7-1-methyl-oxiran als Öl, welches ohne weitere Reinigung in die nächste Stufe eingesetzt werden kann.

In entsprechender Weise und gemäß den allgemeinen Herstellungsangaben wurden die folgenden Verbindungen der Formel (I) erhalten:

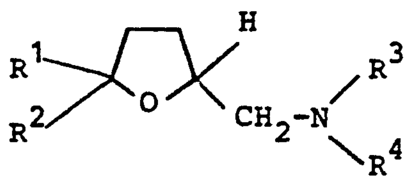

(I)

| Bsp. Nr. | $R^1$ | $R^2$ | 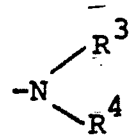 | physikalische Eigenschaften |
|---|---|---|---|---|
| 3 | 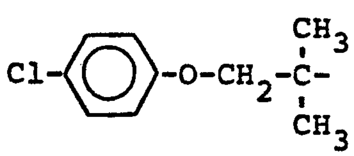 | $CH_3$ | 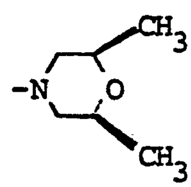 | $n_D^{20}$ 1.5059 (cis-Form) |
| 4 | 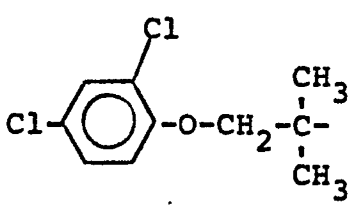 | $CH_3$ | 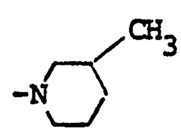 | $n_D^{20}$ 1.5114 |

| Bsp.-Nr. | $R^1$ | $R^2$ | 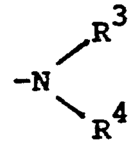 | physikalische Eigenschaften |
|---|---|---|---|---|
| 5 | 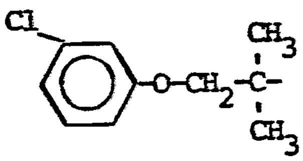 | $CH_3$ | 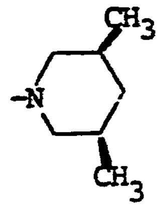 | $n_D^{20}$ 1.5389 (cis-Form) |
| 6 | 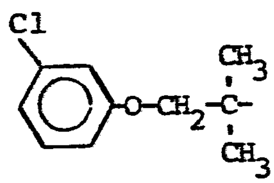 | $CH_3$ | 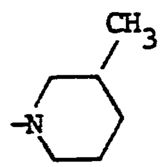 | $n_D^{20}$ 1.5374 |
| 7 | 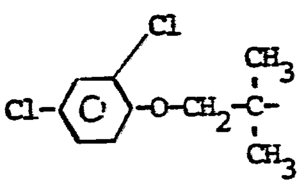 | $CH_3$ | 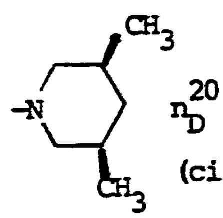 | $n_D^{20}$ 1.5129 (cis-Form) |
| 8 | 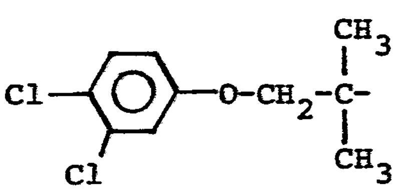 | $CH_3$ | 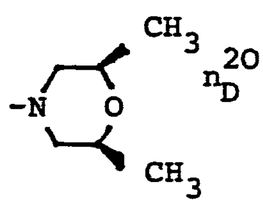 | $n_D^{20}$ 1.5149 (cis-Form) |
| 9 | 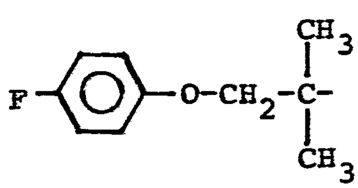 | $CH_3$ | 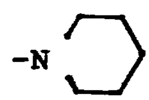 | $n_D^{20}$ 1.4961 |

| Bsp.-Nr. | $R^1$ | $R^2$ | 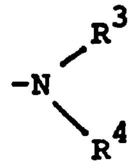 | physikalische Eigenschaften |
|---|---|---|---|---|
| 10 | 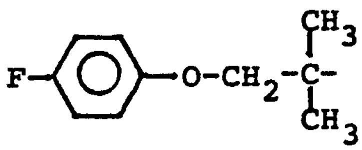 | $CH_3$ | 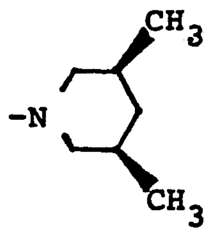 | $n_D^{20}$ 1.4890 (cis-Form) |
| 11 | 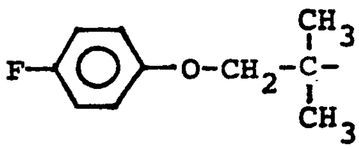 | $CH_3$ | 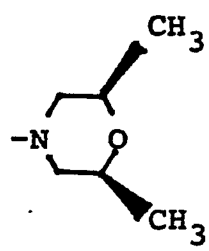 | $n_D^{20}$ 1.4856 (cis-Form) |
| 12 | 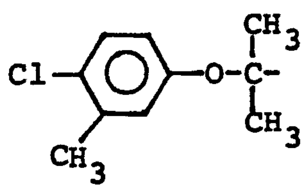 | $CH_3$ | 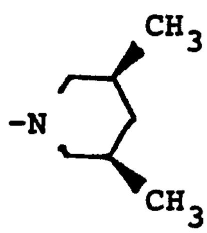 | $n_D^{20}$ 1.5051 (cis-Form) |
| 13 | 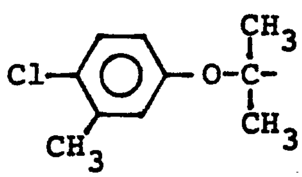 | $CH_3$ | 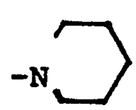 | $n_D^{20}$ 1.5108 |

| Bsp.-Nr. | $R^1$ | $R^2$ | 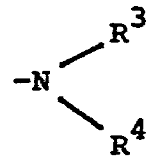 | physikalische Eigenschaften |
|---|---|---|---|---|
| 14 | 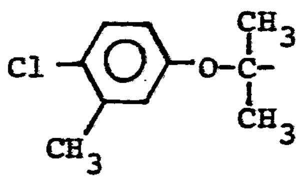 | $CH_3$ | 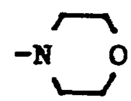 | $n_D^{20}$ 1.5139 |
| 15 | 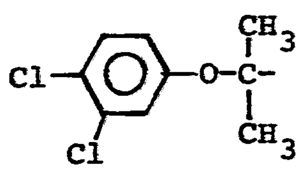 | $CH_3$ | 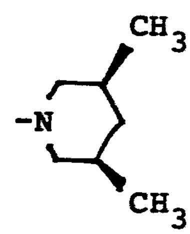 | $n_D^{20}$ 1.5162 (cis-Form) |
| 16 | 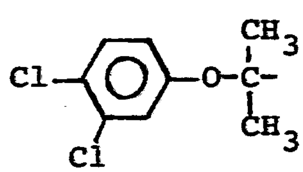 | $CH_3$ | 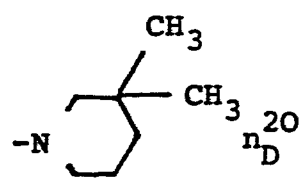 | $n_D^{20}$ 1.5116 |
| 17 | 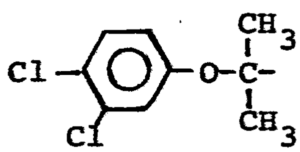 | $CH_3$ | 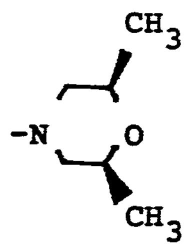 | $n_D^{20}$ 1.5229 (cis-Form) |

## Anwendungsbeispiele

In den folgenden Anwendungsbeispielen werden die nachstehend aufgeführten Verbindungen als Vergleichssubstanzen eingesetzt:

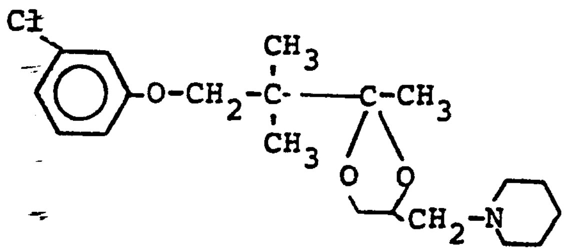

(A)

2-[1-(3-Chlorphenoxy)-2-methylprop-2-yl]-2-methyl-4-(3-methylpiperidin-1-ylmethyl)-1,3-dioxolan

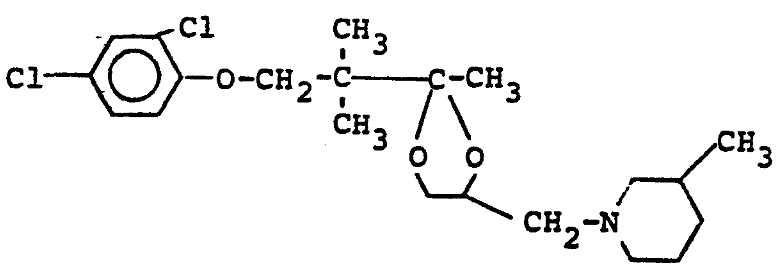

(B)

2-[1-(2,4-Dichlorphenoxy)-2-methylprop-2-yl]-2-methyl-4-(3-methylpiperidin-1-ylmethyl)-1,3-dioxolan

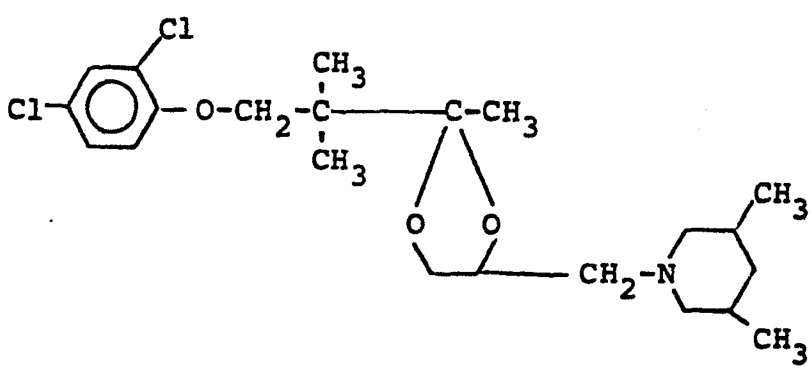

(C)

0158922

2-/1-(2,4-Dichlorphenoxy)-2-methylprop-2-yl7-2-methyl-4-(3,5-dimethylpiperidin-1-ylmethyl)-1,3-dioxolan

(alle bekannt aus DE-OS 3 305 769).

Beispiel A

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 4, 5, 6 und 7.

Beispiel B

Phytophthora-Test (Tomate) /protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1 und 3.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 2 und 4.

Beispiel D

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 2, 3 und 5.

**Beispiel E**

**Wuchsbeeinflussung bei Zuckerrüben**

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Eine deutliche Wirksamkeit zeigt in diesem Test z.B. die Verbindung gemäß Herstellungsbeispiel 1.

Patentansprüche

1. Tetrahydrofuran-2-ylmethylamine der Formel (I)

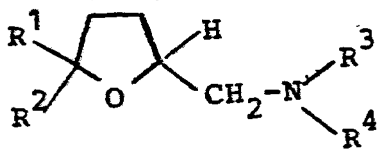

(I)

in welcher

$R^1$ für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes - Phenyl, Phenoxy oder Phenylthio substituiertes Alkyl steht,

$R^2$ für Wasserstoff oder Methyl steht,

$R^3$ für Alkyl steht und

$R^4$ für Alkyl, Alkenyl, Alkinyl oder gegebenenfalls substituiertes Aralkyl steht oder

$R^3$ und $R^4$ gemeinsam mit dem Stickstoffatom, an das sie gebunden sind, für einen gegebenenfalls substituierten gesättigten Heterocyclus stehen, der weitere Heteroatome enthalten kann, ausgenommen die Verbindung, in der $R^3$ und $R^4$ beide

für Methyl stehen, $R^1$ für unsubstituiertes Phenyl und $R^2$ gleichzeitig für Wasserstoff stehen,

sowie deren pflanzenverträgliche Säureadditionssalze und Metallsalzkomplexe.

2. Tetrahydrofuran-2-ylmethylamine gemäß Anspruch 1, wobei in der Formel (I)

$R^1$ für gegebenenfalls einfach oder mehrfach, gleich oder verschieden substituiertes Naphtyhl steht, wobei als Substituenten in Frage kommen: Hydroxy, Halogen, jeweils geradkettiges oder verzweigtes Alkyl, Alkoxy, Alkenyloxy, Alkinyloxy oder Alkanoyloxy mit je bis zu 4 Kohlenstoffatomen in den Alkylteilen; weiterhin für jeweils gegebenenfalls einfach oder mehrfach, gleich oder verschieden durch geradkettiges oder verzweigtes Alkyl mit 1 bis 4 Kohlenstoffatomen substituiertes Cycloalkyl oder Cycloalkenyl mit je 3 bis 7 Kohlenstoffatomen steht; für gegebenenfalls einfach oder mehrfach, gleich oder verschieden substituiertes Phenyl oder für im Phenylkern gegebenenfalls einfach oder mehrfach, gleich oder verschieden substituiertes Phenylalkyl, Phenoxyalkyl oder Phenylthioalkyl mit jeweils bis zu 8 Kohlenstoffatomen im geradkettigen oder verzweigten Alkylteil steht, wobei als Phenylsubstituenten

jeweils in Frage kommen: Hydroxy, Halogen, Cyano, Nitro, jeweils geradkettiges oder verzweigtes Alkyl, Alkoxy, Alkenyloxy, Alkinyloxy und Alkylthio mit jeweils bis zu 4 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Cycloalkyl mit 5 bis 7 Kohlenstoffatomen, geradkettiges oder verzweigtes Alkoxycarbonyl bzw. Alkanoyloxy mit jeweils bis zu 4 Kohlenstoffatomen im Alkylteil, gegebenenfalls durch Halogen, oder durch geradkettiges oder verzweigtes Alkyl mit 1 bis 4 Kohlenstoffatomen substituiertes Phenyl oder Phenoxy, oder der Rest R-O-N=CH-, wobei R jeweils für geradkettiges oder verzweigtes Alkyl, Alkenyl oder Alkinyl mit bis zu 4 Kohlenstoffatomen steht,

$R^2$ für Wasserstoff oder Methyl steht,

$R^3$ für geradkettiges oder verzweigtes Alkyl mit 1 bis 4 Kohlenstoffatomen steht und

$R^4$ für geradkettiges oder verzweigtes Alkyl mit 1 bis 6 Kohlenstoffatomen, für geradkettiges oder verzweigtes Alkenyl oder Alkinyl mit jeweils bis zu 6 Kohlenstoffatomen oder für gegebenenfalls einfach oder mehrfach, gleich oder verschieden substituiertes Aralkyl mit 1 oder

2 Kohlenstoffatomen im Alkylteil und 6 bis 10 Kohlenstoffatomen im Arylteil steht, wobei als Arylsubstituenten jeweils in Frage kommen: Halogen, geradkettiges oder verzweigtes Alkyl mit 1 bis 4 Kohlenstoffatomen oder Halogenalkyl mit 1 oder 2 Kohlenstoffatomen und 1 bis 5 gleichen oder verschiedenen Halogenatomen,

$R^3$ und $R^4$ zusammen mit dem Stickstoffatom, an welches sie gebunden sind, für einen gegebenenfalls einfach oder mehrfach, gleich oder verschieden substituierten 5- bis 7-gliedrigen gesättigten Heterocyclus mit 1 bis 3 Heteroatomen stehen, wobei als Substituenten in Frage kommen: geradkettiges oder verzweigtes Alkyl mit 1 bis 4 Kohlenstoffatomen, geradkettiges oder verzweigtes Alkoxycarbonyl mit bis zu 5 Kohlenstoffatomen, Phenyl, Hydroxymethyl sowie die R'-CO-O-$CH_2$-Gruppe, wobei R' für geradkettiges oder verzweigtes Alkyl, Alkoxy oder Alkylamino bzw. Dialkylamino mit jeweils 1 bis 6 Kohlenstoffatomen in den einzelnen Alkylteilen, für geradkettiges oder verzweigtes Alkoxyalkyl mit jeweils 1 bis 6 Kohlenstoffatomen in beiden Alkylteilen steht, ausgenommen die Verbindung, in der $R^3$ und $R^4$ beide für Methyl, $R^1$ für unsubstituiertes Phenyl und $R^2$ gleichzeitig für Wasserstoff stehen.

3. Tetrahydrofuran-2-ylmethylamine gemäß Anspruch 1, wobei in der Formel (I)

$R^1$ für gegebenenfalls ein- bis dreifach, gleich oder verschieden substituiertes Naphthyl steht, wobei als Substituenten in Frage kommen: Fluor, Chlor, Brom, Hydroxy, Methyl, Ethyl, Methoxy, Ethoxy, Allyloxy, Propargyloxy und Acetyloxy,

weiterhin für jeweils gegebenenfalls ein- bis dreifach, gleich oder verschieden durch Methyl oder Ethyl substituiertes Cycloalkyl oder Cycloalkenyl mit 5 bis 7 Kohlenstoffatomen, für gegebenenfalls ein- bis dreifach, gleich oder verschieden substituiertes Phenyl oder für einen im Phenylkern gegebenenfalls ein- bis dreifach, gleich oder verschieden substituierten Rest der Formel

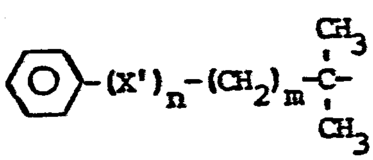 , 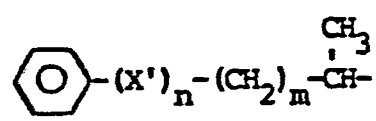 ,

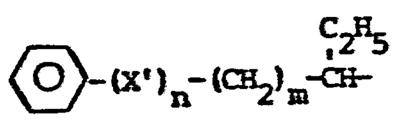 oder 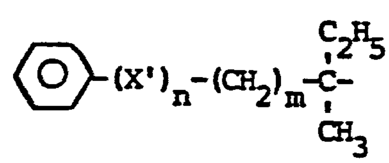

steht, wobei als Phenylsubstituenten jeweils in Frage kommen: Hydroxy, Fluor, Chlor, Brom,

Cyano, Nitro, Methyl, Methoxy, Methylthio, Ethyl, Ethoxy, Ethylthio, n- und i-Propyl, Isopropyloxy, n-, i-, s- und t-Butyl, Allyloxy, Propargyloxy, Trifluormethyl, Trifluormethoxy, Trifluormethylthio, Cyclohexyl, Methoxycarbonyl, Ethoxycarbonyl, Acetyloxy, gegebenenfalls durch Fluor, Chlor oder Methyl substituiertes Phenyl oder Phenoxy, sowie der Rest R-O-N=CH-, wobei R jeweils für Methyl, Ethyl, n- und i-Propyl, n-, i-, s- und t-Butyl, Allyl und Propargyl steht,

wobei in allen oben formelmäßig dargestellten Resten X' jeweils für Sauerstoff oder Schwefel, n für 0 oder 1 und m ebenfalls für 0 oder 1 stehen,

$R^2$ für Wasserstoff oder Methyl steht,

$R^3$ für Methyl, Ethyl, n- und i-Propyl steht und

$R^4$ für Methyl, Ethyl, n- und i-Propyl, n-, i-, s- und t-Butyl, Neopentyl, Allyl, 2-Butenyl, 3-Methyl-2-butenyl, Propargyl sowie für gegebenenfalls ein- bis dreifach, gleich oder verschieden durch Fluor, Chlor, Methyl oder Trifluormethyl substituiertes Benzyl steht oder

$R^3$ und $R^4$ zusammen mit dem Stickstoffatom, an welches sie gebunden sind, für jeweils gegebenenfalls ein- bis dreifach, gleich oder verschieden substituiertes 1-Pyrrolidinyl, 1-Piperidinyl, 1-Piperazinyl, 4-Morpholinyl oder 1-Perhydroazepinyl stehen, wobei als Substituenten genannt seien: Methyl, Ethyl, n- und i-Propyl, Phenyl, Hydroxymethyl, Acetyloxymethyl, Methoxycarbonyloxymethyl, Dimethylaminocarbonyloxymethyl, Diethylaminocarbonyloxymethyl oder Methoxyacetyloxymethyl, ausgenommen die Verbindung, in der $R^3$ und $R^4$ beide für Methyl, $R^1$ für unsubstituiertes Phenyl und $R^2$ gleichzeitig für Wasserstoff stehen.

4. Verfahren zur Herstellung von Tetrahydrofuran-2-yl-methylaminen der allgemeinen Formel (I)

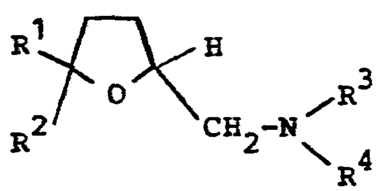

(I)

in welcher

$R^1$ für gegebenenfalls substituiertes Naphthyl, für gegebenenfalls substituiertes Cycloalkyl und Cycloalkenyl, für gegebenenfalls substituiertes Phenyl oder für durch - jeweils gegebenenfalls substituiertes - Phenyl, Phenoxy oder Phenylthio, substituiertes Alkyl steht,

$R^2$ für Wasserstoff oder Methyl steht,

$R^3$ für Alkyl steht und

$R^4$ für Alkyl, Alkenyl, Alkinyl oder gegebenenfalls substituiertes Aralkyl steht oder

$R^3$ und $R^4$ gemeinsam mit dem Stickstoff, an das sie gebunden sind, für einen gegebenenfalls substituierten gesättigten Heterocyclus stehen, der weitere Heteroatome enthalten kann, ausgenommen die Verbindung, in der $R^3$ und $R^4$ jeweils für Methyl, $R^1$ für unsubstituiertes Phenyl und $R^2$ für Wasserstoff stehen,

sowie deren pflanzenverträglichen Säureadditionssalze und Metallsalzkomplexe, dadurch gekennzeichnet, daß man Tetrahydrofuranderivate der Formel (II)

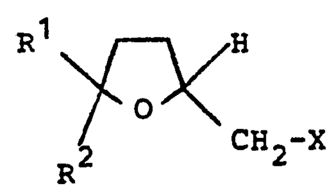

(II)

in welcher

$R^1$ und $R^2$ die oben angegebene Bedeutung haben und

X für eine elektronenanziehende Abgangsgruppierung steht,

mit Aminen der allgemeinen Formel (III)

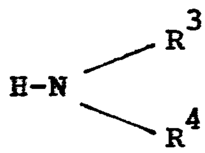

(III)

in welcher

$R^3$ und $R^4$ die oben angegebene Bedeutung haben,

gegebenenfalls in Gegenwart eines Verdünnungsmittels, gegebenenfalls in Gegenwart eines Katalysators und gegebenenfalls in Gegenwart eines Säurebindemittels umsetzt, und gegebenenfalls anschließend eine Säure oder ein Metallsalz addiert.

5. Pflanzenschutzmittel, gekennzeichnet durch einen Gehalt an mindestens einem Tetrahydrofuran-2-ylmethylamin der Formel (I) nach den Ansprüchen 1 und 4.

6. Fungizide und das Pflanzenwachstum regulierende Mittel, gekennzeichnet durch einen Gehalt an mindestens einem Tetrahydrofuran-2-ylmethylamin der Formel (I) nach den Ansprüchen 1 und 4.

7. Verfahren zur Bekämpfung von Pilzen und zur Regulierung des Pflanzenwachstums, dadurch gekennzeichnet, daß man Tetrahydrofuran-2-ylmethylamine der Formel (I) nach den Ansprüchen 1 und 4 auf Pilze oder Pflanzen oder ihren Lebensraum einwirken läßt.

8. Verwendung von Tetrahydrofuran-2-ylmethylaminen der Formel (I) nach den Ansprüchen 1 und 4 als Pflanzenschutzmittel.

9. Verwendung von Tetrahydrofuran-2-ylmethylaminen der Formel (I) nach den Ansprüchen 1 und 4 zur Bekämpfung von Pilzen und zur Regulierung des Pflanzenwachstums.

10. Verfahren zur Herstellung von Pflanzenschutzmitteln, dadurch gekennzeichnet, daß man Tetrahydrofuran-2-ylmethylamine der Formel (I) nach den Ansprüchen 1 und 4 mit Streckmitteln und/oder oberflächenaktiven Mitteln vermischt.